Europäisches Patentamt

European Patent Office

Office européen des brevets

(11) Numéro de publication: **0 211 713**

**A1**

## (12) DEMANDE DE BREVET EUROPEEN

(21) Numéro de dépôt: 86401450.1

(51) Int. Cl.⁴: **C07D 307/50**

(22) Date de dépôt: 30.06.86

(30) Priorité: 05.07.85 FR 8510345

(43) Date de publication de la demande:
25.02.87 Bulletin 87/09

(84) Etats contractants désignés:
AT CH DE GB IT LI SE

(71) Demandeur: COFINPAR S.A.
Berkeley Building 20-24 rue du Capitaine Guynemer
F-92081 Paris La Défense 2 Cédex 19(FR)
Demandeur: Maurice, Pierre Marie Paul
99 rue de Prony
F-75017 Paris(FR)

(72) Inventeur: Maurice, Pierre Marie Paul
99 rue de Prony
F-75017 Paris(FR)

(74) Mandataire: Durand, Yves Armand Louis et al
Cabinet Z. Weinstein 20, Avenue de Friedland
F-75008 Paris(FR)

(54) Procédé et installation de fabrication de furfural à partir de solutions aqueuses liquides de xyloses, de préférence à partir d'une liqueur noire résiduelle de fabrication de pâte à papier ou de pâte textile.

(57) L'invention concerne un procédé et une installation de fabrication de furfural à partir de solutions aqueuses liquides de xyloses, en particulier à partir de pâtes papetières ou textiles.

L'installation comprend un réacteur d'hydrolyse sous pression et est caractérisée en ce qu'elle comprend au moins deux dispositifs de détente et de séparation (6, 16, 18) au moins un dispositif de détente et de séparation intermédiaire (6 ; 16) et un dispositif de détente ultime (18) et de séparation ; un échangeur de chaleur formant évaporateur (12, 14) étant disposé entre deux dispositifs de détente successifs, comportant un circuit de fluide chauffant et un circuit de fluide réfrigérant, des vapeurs produites par le dispositif de détente amont étant introduites dans le circuit de fluide chauffant pour réaliser un échange thermique indirect avec le liquide sortant du dispositif de détente aval introduit dans le circuit de fluide réfrigérant, les vapeurs condensées (CV) étant envoyées en phase liquide à ladite colonne de distillation tandis que les vapeurs produites par au moins une détente intermédiaire (16) et/ou par la détente ultime (18) sont utilisées au moins en partie pour surconcentrer la solution et/ou pour effectuer un échange thermique avec les condensats introduits dans la colonne (8) pour les distiller.

Selon l'invention, on simplifie la conception de l'installation tout en améliorant le rendement thermique global.

## "Procédé et installation de fabrication de furfural à partir de solutions aqueuses liquides de xyloses de préférence à partir d'une liqueur noire résiduelle de fabrication de pâte à papier ou de pâte textile"

L'invention concerne un procédé et une installation de fabrication de furfural à partir de solutions aqueuses liquides de xyloses, de préférence une liqueur noire résiduelle de fabrication de pâte à papier ou de pâte textile.

On connaît à partir du document FR-A-1 510 864 SICAMIE un procédé et une installation de fabrication de furfural à partir des liqueurs résiduaires de cuisson du bois.

Le procédé décrit comprend la séparation préalable du furfural de la liqueur résiduelle par une première détente, les vapeurs produites étant condensées et ensuite distillées et rectifiées.

La liqueur ainsi débarassée du furfural est ensuite hydrolysée par chauffage sous pression en milieu acide pour réaliser une hydrolyse acide. La pression d'hydrolyse est élevée, par exemple de 50 kg/cm² (voir exemples 1 et 2).

Cette liqueur hydrolysée sous pression est soumise à une détente entraînant le furfural dans les vapeurs formées. Les vapeurs sont condensées et la récupération du furfural est effectuée par distillation et rectification (voir page 1, colonne de gauche, troisième paragraphe complet).

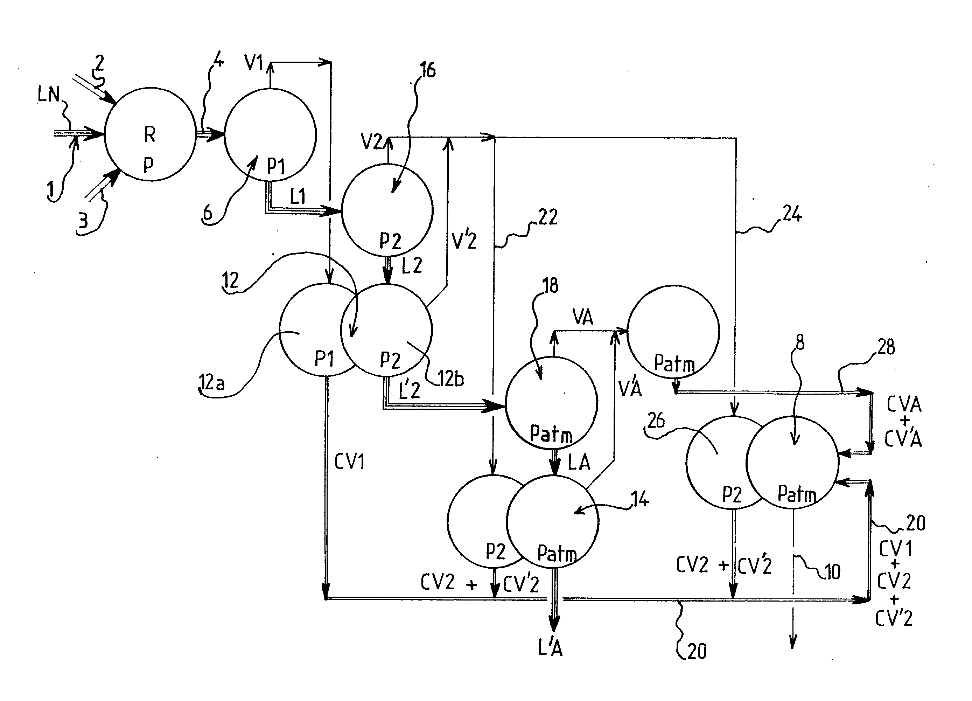

La liqueur hydrolysée détendue est indépendamment concentrée dans plusieurs colonnes de concentration 4, 5, 9, figure 1. Selon la variante de la figure 2, après la première détente effectuant la séparation préalable du furfural déjà contenu dans la liqueur résiduaire de cuisson du bois, cette liqueur détendue est concentrée indépendamment dans deux colonnes de concentration 4, 5 avant que cette liqueur soit soumise à la réaction d'hydrolyse précitée en étant recomprimée à une pression de 50 kg/cm², dans le réacteur 6. Selon cette variante, on effectue ensuite une détente avant de procéder à une concentration indépendante dans la colonne de concentration 9.

Ce procédé et l'installation concomittante permettent de récupérer le furfural avec un rendement correct compte tenu qu'il y aurait lieu de faire un stripping pour récupérer tout le furfural.

En outre, les procédés et l'installation décrits dans ce document sont d'un coût énergétique très élevé car ils exigent tout d'abord une recompression après détente et utilisent des colonnes de concentration indépendantes.

Ceci complique en outre l'installation et en augmente radicalement le coût.

On a proposé d'autres solutions dans les documents suivants : EP-A-36 406, EP-A-38 317 et FR-2411184.

Selon le document EP-A-36 406, on réalise une extraction avec un oxyde de trialcoyle phosphine insoluble dans l'eau, le milieu d'extraction chargé est ensuite distillé de manière à séparer les substances organiques pures, et notamment le furfural.

Le document FR-2411184 est aussi relatif à une extraction par solvant. L'extraction par solvant complique le procédé et est d'un coût relativement élevé.

Dans le second document EP-A-38 317, on réalise un échange thermique à contre-courant de la liqueur avant sa pénétration dans le réacteur R, avec la liqueur chauffée hydrolysée sortant du réacteur.

Cette liqueur sortant du réacteur ainsi refroidi est ensuite distillée.

D'autre part, la récupération de la chaleur de la liqueur sortant du réacteur par échange de chaleur avec la liqueur entrant ou avec tout autre fluide est d'un faible intérêt pratique par suite de l'encrassement très rapide des surfaces d'échange.

D'autre part, la liqueur résiduaire débarrassée de furfural présente une concentration identique à la concentration de la liqueur entrant alors que l'on recherche l'obtention d'une liqueur la plus concentrée possible. En effet, dans le cas de la pâte à papier, la liqueur est ensuite brûlée et l'augmentation de concentration entraîne la production de calories supplémentaires.

On retiendra d'ailleurs que c'est cette solution qui est préconisée par le document FR-A-1 510 864.

La présente invention a donc pour but de résoudre le nouveau problème technique consistant en la fourniture d'un procédé et d'une installation simplifiés pour la fabrication de furfural, réalisant simultanément une concentration de la liqueur noire résiduelle de fabrication de pâte à papier, de manière plus générale de solutions aqueuses de xylose, avec une augmentation du rendement thermique global et par conséquent une diminution radicale du coût de fonctionnement d'un tel procédé et d'une telle installation.

Ce nouveau problème technique est résolu pour la première fois par la présente invention.

Ainsi, selon un premier aspect de l'invention, on fournit un procédé de fabrication de furfural à partir de solutions aqueuses liquides de xyloses, de préférence une liqueur noire résiduelle de fabrication de pâte à papier, ou de préférence de pâte textile, comprenant l'introduction sous pression élevée appropriée de ladite solution aqueuse de xylose dans un réacteur, le chauffage de ladite solution aqueuse et éventuellement son acidification dans ledit réacteur, et le traitement de la solution aqueuse contenant le furfural produit pour en extraire le furfural, ledit traitement comprenant une détente de la solution aqueuse de manière à obtenir des vapeurs contenant le furfural, et de la solution liquide résiduaire, lesdites vapeurs étant ensuite condensées et les condensats distillés pour récupérer le furfural, ladite solution liquide étant concentrée et les vapeurs obtenues lors de la concentration étant condensées puis également distillées, caractérisé en ce que, en vue de simplifier le procédé tout en obtenant une solution aqueuse concentrée avec un excellent rendement thermique global, on procède à au moins deux détentes de la solution liquide sortant du réacteur, contenant du furfural ; au moins une détente intermédiaire et une détente ultime, les vapeurs produites par chaque détente intermédiaire étant utilisées au moins en partie pour concentrer la solution liquide restante après la détente suivante, en obtenant ainsi des vapeurs condensées que l'on soumet à ladite distillation, les vapeurs produites par au moins une détente intermédiaire et/ou la détente ultime étant utilisées au moins en partie pour surconcentrer la solution liquide et/ou pour chauffer et distiller lesdites vapeurs condensées.

Selon une caractéristique préférée de ce procédé, on réalise au moins trois détentes successives.

Selon une caractéristique particulière, les vapeurs produites sont utilisées en partie pour surconcentrer la solution liquide et en partie pour chauffer et distiller les vapeurs précédemment condensées.

La présente invention concerne également selon un autre de ses aspects, une installation de fabrication de furfural à partir de solutions aqueuses liquides de xyloses, comprenant un réacteur pourvu d'au moins une conduite d'amenée de ladite solution aqueuse de xylose sous une pression P appropriée, éventuellement au moins une conduite d'amenée d'agent acidifiant sous pression, au moins une conduite d'amenée de fluide chauffant, de préférence de la vapeur d'eau sous pression, au moins une conduite d'évacuation de ladite solution aqueuse contenant le furfural produit, à la pression P, reliant ledit réacteur à un dispositif de détente et de séparation, abaissant la pression et séparant les vapeurs produites lors de la détente contenant du furfural, de la solution liquide appauvrie en furfural, une colonne de distillation desdites vapeurs produites qui ont été condensées, pour récupérer du furfural purifié, et au moins un appareil de concentration de la solution liquide pour obtenir une solution liquide concentrée, les vapeurs produites par la concentration contenant du furfural supplémentaire étant envoyées à ladite colonne de distillation après condensation, caractérisée en ce qu'elle comprend au moins deux dispositifs de détente et de séparation, au moins un dispositif de détente et de séparation intermédiaire et un dispositif de détente ultime et de séparation ; un échangeur de chaleur formant évaporateur étant disposé entre deux dispositifs de détente successifs, comportant un circuit de fluide chauffant et un circuit de fluides réfrigérants, les vapeurs produites par le dispositif de détente amont étant introduites dans le circuit de fluide chauffant pour réaliser un échange thermique indirect avec le liquide sortant du dispositif de détente aval introduit dans le circuit de fluide réfrigérant, les vapeurs condensées étant envoyées en phase liquide à ladite colonne de distillation tandis que les vapeurs produites par au moins une détente intermédiaire et/ou par la détente ultime sont utilisées au moins en partie pour surconcentrer la solution liquide et/ou pour effectuer un échange thermique avec les condensats introduits dans la colonne pour les distiller.

Selon un mode de réalisation préféré de l'installation selon l'invention, on utilise au moins trois dispositifs de détente.

D'autre part, selon une caractéristique préférée de l'installation selon l'invention, les dispositifs de détente sont situés à un niveau supérieur par rapport à l'échangeur de chaleur pour réaliser une alimentation par gravité. De même les sorties de condensat seront situées à un niveau supérieur au niveau d'introduction de ceux-ci dans la colonne de distillation pour assurer une alimination par gravité.

On constate ainsi que selon la présente invention, l'échangeur de chaleur constitue un évaporateur et constitue donc en même temps l'appareil de concentration antérieurement utilisé qui est, selon l'invention, combiné d'une manière entièrement nouvelle pour simplifier la conception de l'installation et du procédé concomittant et pour diminuer de manière radicale la consommation. énergétique en améliorant le rendement thermique global. En effet, on aboutit à une récupération du furfural purifié après distillation d'un condensat, sans apport de calories autres que les calories qui ont été utilisées pour réaliser la fabrication du furfural dans le réacteur tout en obtenant une solution liquide hautement concentrée par rapport à l'entrée.

Dans le cas préféré de l'application à l'utilisation d'une liqueur noire résiduelle de fabrication de pâte à papier, ou de pâte textile où les liqueurs sont brûlées, cette augmentation de concentration entraîne la production de calories supplémentaires.

Enfin, par le positionnement des sorties de condensat à un niveau supérieur assurant l'introduction de ces condensats dans la colonne de distillation, on obtient un fonctionnement de l'installation sans pompe de circulation par simple écoulement par gravité.

D'autres buts, caractéristiques et avantages de l'invention apparaîtront clairement à la lumière de la description explicative qui va suivre faite en référence à la figure unique annexée représentant un schéma de principe de l'installation selon l'invention, permettant la mise en oeuvre du procédé selon l'invention précédemment décrite. Le circuit ═══⟹ représente le trajet suivi par la solution liquide ; le circuit ═══⟹ représente le trajet suivi par les condensats et le circuit ⟶représente le trajet suivi par les vapeurs.

En référence à la figure unique annexée, une installation selon l'invention de fabrication de furfural à partir d'une solution aqueuse liquide de xylose, de préférence une liqueur noire (LN) résiduelle de fabrication de pâte à papier, ou de préférence de pâte textile par exemple à partir de bois feuillus ou de végétaux annuels, avantageusement par un procédé acide, comprend un réacteur R pourvu d'au moins une conduite 1 d'amenée de ladite solution aqueuse liquide (LN) sous pression P appropriée ; éventuellement au moins une conduite d'amenée d'agent acidifiant 2, sous pression, au moins une conduite d'amenée de fluide chauffant 3, de préférence de la vapeur d'eau sous pression, au moins une conduite d'évacuation 4 de la solution aqueuse acidifiée contenant le furfural produit, à la pression P, reliant ledit réacteur R à un dispositif de détente et de séparation représenté par le numéro de référence général 6, abaissant la pression et séparant les vapeurs produites lors de la détente contenant principalement du furfural (V₁) de la solution liquide appauvrie en furfural L1, une colonne de distillation, désignée par le repère général 8, desdites vapeurs produites qui ont été condensées pour récupérer du furfural distillé, purifié par la conduite 10, normalement sous forme azéotrope qui peut ensuite être rectifié pour obtenir du furfural rectifié.

L'installation comprend au moins un appareil de concentration 12, 14 de la solution liquide pour obtenir une solution liquide concentrée, les vapeurs produites par la concentration (désignée dans la figure unique V'2, V'A) contenant du furfural supplémentaire étant envoyées à la colonne de distillation 8.

Selon la présente invention, l'installation comprend au moins deux dispositifs de détente ou de séparation, au moins un dispositif de détente et de séparation intermédiaire désigné par le numéro de référence 6, 16 et un dispositif de détente et de séparation ultime 18.

Ainsi, dans la figure unique annexée qui représente le mode de réalisation actuellement préféré de l'installation, au moins trois dispositifs de détente 6, 16 et 18 sont présents.

Selon la présente invention, les vapeurs produites V1, V2 par chaque détente intermédiaire 6, 16, respectivement, sont utilisées au moins en partie pour concentrer la solution liquide restante après la détente suivante.

Pour ce faire, selon l'invention, on prévoit au moins un échangeur de chaleur formant en même temps l'appareil d'évaporation précité (12) coopérant avec deux dispositifs de détente successifs tels que 6, 16 comprenant un circuit de fluides chauffants 12a et un circuit de fluide réfrigérant 12b. Les vapeurs produites par le dispositif de détente situé en amont (V1) sont introduites dans le circuit de fluide chauffant 12a à la pression P1, et réalisant un échange thermique avec le liquide sortant du dispositif de détente aval (L2) introduit à la pression P2 dans le circuit de fluide réfrigérant 12b.

La pression P1 étant supérieure à la pression P2, tandis que la température des vapeurs V1 étant nettement plus élevée que la température du liquide L2, on aboutit à une condensation des vapeurs V1 en formant ainsi des condensats CV1 qui sont envoyés en phase liquide à la colonne de distillation 8 par la conduite de collecte des condensats 20 tandis que les vapeurs produites V'2 par l'échange thermique sont utilisées pour effectuer soit une surconcentration de la solution liquide LA sortant de la détente ultime dans l'appareil de détente ultime 18 dans un nouvel échangeur de chaleur 14 par une conduite 22, soit sont envoyées par une conduite en dérivation 24 pour effectuer un échange thermique dans la partie formant bouilleur 26 de la colonne de distillation 8 de manière à chauffer et distiller les condensats introduits notamment par la conduite 20, soit les deux possibilités, comme représenté sur la figure unique annexée.

D'autre part, les vapeurs produites dans la détente ultime dans le dispositif de détente ultime 18, ainsi que les vapeurs produites par le dernier échangeur de chaleur 14, (VA et V'A respectivement, peuvent être soit mélangées aux vapeurs V2 dans la conduite 24 pour chauffer les condensats, en étant également à leur tour condensées en formant des condensats CV2 et CV'2 et ensuite amenées à la colonne de distillation 8 par la conduite 20, soit séparément condensées par un échange thermique avec un fluide réfrigérant quelconque, tel que de l'eau, en étant amenées séparément par une conduite 28 à la colonne de distillation 8.

On donne un exemple d'application du procédé et de l'installation selon l'invention, ci-dessous :

EXEMPLE

Par exemple, on peut traiter 24 tonnes par heure de liqueur noire résiduelle de fabrication de la pâte à papier par le procédé acide, concentrée à 40 % de matières sèches disponibles à la sortie de l'installation de concentration classique, à une température d'environ 100°C.

Cette liqueur noire sera refoulée sous une pression de 26 bars absolus dans le réacteur R par la conduite 1 où elle sera réchauffée à 215°C par injection de vapeur par la conduite 3 à pression égale à 38 bars et température égale à 450°C.

L'ensemble, soit 24 + 3,9 tonnes par heure égale 27,9 tonnes par heure, sera détendu à la pression de 4 bars absolus (P1) et la vaporisation qui en résultera conduira à : 24,5 tonnes/heure de liquide L1 et 3,4 tonnes/heure de vapeur V1 à la pression P1 de 4 bars absolus.

La nouvelle détente intermédiaire dans le dispositif de détente 16 du liquide L1 séparé du premier dispositif de détente 6, à la pression P2 égale à 2 bars entraînera une vaporisation partielle conduisant à 23,76 tonnes par heure de liquide L2 et 0,74 tonne de vapeur V2 à la pression P2 égale à 2 bars absolus.

L'utilisation des 3,4 tonnes par heure de vapeur V1 pour la concentration de 23,76 tonnes par heure de liqueur L2 dans l'échangeur de chaleur 12 conduira à la formation de 3,32 tonnes par heure de vapeur V'2 et de 20,44 tonnes par heure de liquide L'2 à la pression P2 de 2 bars absolus, alors qu'on obtiendra un condensat de vapeur CV1 de 3,4 tonnes par heure alimenté par la conduite 20 à la colonne de distillation 8.

La détente du liquide L'2 depuis la pression intermédiaire P2 égale à 2 bars absolus à la pression atmosphérique dans le dispositif de détente ultime 18 entraînera une nouvelle vaporisation partielle conduisant à 0,52 tonne par heure de vapeur VA et 19,92 tonnes par heure de liquide restant LA à la pression atmosphérique.

Si on désire amener la concentration de la liqueur à environ 50 %, on pourra le faire en consommant une partie des vapeurs disponibles V2 à pression P2 de 2 bars et obtenir 0,94 tonne/heure de vapeur V'A et 18,98 tonnes par heure de liquide résiduaire concentré L'A.

La vapeur disponible à la pression P2 de 2 bars absolus, après prélèvement pour cette surconcentration, reste encore suffisante pour assurer la distillation azéotrope de l'ensemble des vapeurs et condensats chargés de furfural, soit :

-3,40 tonnes par heure de CV1 ;

-0,52 tonne par heure de CVA ;

-0,94 tonne par heure de CV'A ;

-0,74 tonne par heure de CV2 ; et

-3,32 tonnes par heure de CV'2,

soit au total 8,92 tonnes par heure.

Ainsi, avec une liqueur d'origine à 40 % de matières sèches et contenant 30 kg de xyloses par tonne de liqueur, on pourra obtenir à partir de l'azéotrope produit par la colonne de distillation environ 160 kg de furfural par heure, compte tenu du rendement de transformation de xyloses en furfural et du rendement de récupération au cours des vaporisation, concentration et distillation.

Mais on doit souligner que si la concentration en xylose des liqueurs de pâtes papetières est de l'ordre de 30 kg par tonne de liqueur concentrée, elle peut atteindre 120 kg par tonne pour la liqueur de pâte textile. Dans ce cas, avec les mêmes consommations de vapeur on obtiendra une production de furfural de 640 kg par heure.

On observera également que selon l'invention, la seule consommation de vapeur pour aboutir à l'azéotrope est celle de la vapeur injectée au réacteur, soit 3,9 tonnes par heure.

Par ailleurs, la liqueur noire sortant de installation après extraction de furfural, est à une concentration en matières sèches de 50 % au lieu de 40 % à l'entrée. Il en résulte un pouvoir calorifique plus élevé permettant la production d'une quantité de vapeurs accrue de l'ordre de 3 tonnes par heure, si bien que c'est seulement 3,9 -3,0 = 0,9 tonne par heure de vapeur qui est effectivement consommée par la production de furfural.

La consommation spécifique ressort à :

a) 0,9 tonne de vapeur : 0,16 tonne de furfural = 5,6 tonnes de vapeur par tonne de furfural si la concentration en xylose de la liqueur est de 30 kg par tonne ; ou

b) 0,9 tonne de vapeur/0,64 tonne de furfural = 1,4 tonne de vapeurs par tonne de furfural si la concentration en xylose de la liqueur est de 120 kg/tonne.

Or, avec le procédé connu, consistant à opérer la réaction comme précédémment décrit mais à détendre directement à la pression atmosphérique et à procéder ensuite à un stripping de la liqueur noire à la vapeur pour parfaire la récupération du furfural produit, on obtient les mêmes productions de furfural mais avec une consommation de vapeur qui comprend non seulement celle du réchauffage dans le réacteur mais encore celle nécessaire au stripping et celle nécessaire à la distillation, le total étant de l'ordre de 9,6 tonnes de vapeurs par heure.

Les consommations spécifiques deviennent donc :

a) 9,6 : 0,16 = 60 tonnes de vapeur par tonne de fufural si la concentration de la liqueur est de 30 kg de xyloses par tonne ; ou

b) 9,6 : 0,64 = 15 tonnes de vapeur par tonne de furfural si la concentration de la liqueur est de 120 kg de xyloses par tonne.

On voit donc que l'invention permet, par rapport au procédé connu, de diviser par un facteur supérieur à 10 la consommation spécifique de la vapeur qui est un des postes essentiels du prix de revient.

L'invention permet également de réduire de façon très sensible la consommation d'eau de refroidissement puisqu'au lieu d'avoir à condenser avec cette eau, l'alimentation de la colonne azéotrope, environ 6 tonnes par heure de vapeur, on a condensé au plus VA (0,52 tonne par heure) et V'A (0,94 tonne par heure) soit environ 1,5 tonne par heure et donc 4 fois moins.

On observera ainsi que les pressions de détente $P_1$ (4 bars) $P_2$ (2 bars) et $P_3$ (Patm) sont choisies de telle sorte que l'écoulement des liquides s'effectue sans pompe de reprise sous l'effet des écarts de pression.

On comprend ainsi que l'invention permet d'obtenir tous les avantages précédemment énoncés.

On notera que la distillation peut également être effectuée sous vide partiel et dans ce cas le rebouilleur 26 peut être alimenté en vapeurs à une pression voisine de la pression atmosphérique ce qui entraîne une deuxième détente ($P_2$) à cette pression et donc l'emploi de seulement deux détentes conformément à ce qui a étéprécédemment énoncé.

On obtient le même rendement thermique avec simplement une liqueur résiduaire (L'A) un peu moins concentrée.

Egalement on prévoit chaque pression de détente et en particulier la pression de détente ultime à une valeur telle qu'il existe un écart de pression suffisant pour que le liquide puisse circuler sans pompe.

**Revendications**

1.-Procédé de fabrication de furfural à partir de solutions aqueuses liquides de xyloses, de préférence une liqueur noire résiduelle de fabrication de pâte à papier, ou de pâte textile, comprenant l'introduction sous pression élevée appropriée de ladite solution aqueuse de xyloses dans un réacteur, le chauffage desdites solutions aqueuses éventuellement son acidification dans ledit réacteur, et le traitement de la solution aqueuse contenant le furfural produit pour en extraire le furfural, ledit traitement comprenant une détente de la solution aqueuse de manière à obtenir des vapeurs contenant le furfural, et de la solution liquide résiduaire, lesdites vapeurs étant ensuite condensées et les condensats distillés pour récupérer le furfural, ladite solution liquide étant concentrée et les vapeurs obtenues lors de la concentration étant condensées puis également distillées, caractérisé en ce que, en vue de simplifier le procédé tout en obtenant une solution aqueuse concentrée avec un excellent rendement thermique global, on procède à au moins deux détentes de la solution liquide sortant du réacteur, contenant du furfural ; au moins une détente intermédiaire et une détente ultime, les vapeurs produites par chaque détente intermédiaire étant utilisées au moins en partie pour concentrer la solution liquide restante après la détente suivante, en obtenant ainsi des vapeurs condensées que l'on soumet à ladite distillation, les vapeurs produites par au moins une détente intermédiaire et/ou par la détente ultime étant utilisées au moins en partie pour surconcentrer la solution liquide et/ou pour chauffer et distiller lesdites vapeurs condensées.

2.-Procédé selon la revendication 1, caractérisé en ce qu'on réalise au moins trois détentes successives.

3.-Procédé selon la revendication 1 ou 2, caractérisé en ce que les vapeurs produites sont utilisées en partie pour surconcentrer la solution liquide et en partie pour chauffer et distiller les vapeurs précédemment condensées.

4.-Installation de fabrication de furfural à partir de solutions aqueuses liquides de xyloses, comprenant un réacteur pourvu d'au moins une conduite d'amenée de ladite solution aqueuse de xyloses sous une pression P appropriée, éventuellement au moins une conduite d'amenée d'agent acidifiant sous pression, au moins une conduite d'amenée de fluide chauffant, de préférence de la vapeur d'eau sous pression, au moins une conduite d'évacuation de ladite solution aqueuse contenant le furfural produit, à la pression P, reliant ledit réacteur à un dispositif de détente et de séparation, abaissant la pression et séparant les vapeurs produites lors de la détente contenant du furfural, de la solution liquide appauvrie en furfural, une colonne de distillation desdites. vapeurs produites qui ont été condensées, pour récupérer du furfural purifié, et au moins un appareil de concentration de la solution liquide pour obtenir une solution liquide concentrée, les vapeurs produites par la concentration contenant du furfural supplémentaire étant envoyées à ladite colonne de distillation après condensation, caractérisée en ce qu'elle comprend au moins deux dispositifs de détente et de séparation (6, 16, 18) au moins un dispositif de détente et de séparation intermédiaire (6, 16) et un dispositif de détente ultime et de séparation (18); un échangeur de chaleur (12 ; 14) formant évaporateur étant disposé entre deux dispositifs de détente successifs, comportant un circuit de fluide chauffant et un circuit de fluide réfrigérant, des vapeurs produites par le dispositif de détente amont (6) étant introduites dans le circuit de fluide chauffant (12a) pour réaliser un échange thermique indirect avec le liquide sortant du dispositif de détente aval (L2) introduit dans le circuit de fluide réfrigérant (12b), les vapeurs condensées (CV1) étant envoyées en phase liquide à ladite colonne de distillation (8), tandis que les vapeurs produites par au moins une détente intermédiaire (V2) et/ou par la détente ultime (VA) sont utilisés au moins en partie pour surconcentrer la solution liquide et/ou pour effectuer un échange thermique avec les condensats introduits dans la colonne (8) pour les distiller.

5.-Installation selon la revendication 4, caractérisée en ce qu'elle comprend au moins trois dispositifs de détente (6, 16, 18) et une séparation.

6.-Installation selon l'une des revendications 3 à 5, caractérisée en ce que les dispositifs de détente (6, 16, 18 sont situés à un niveau supérieur par rapport à l'échangeur de chaleur (12, 14, 26) pour réaliser une alimentation par gravité.

7.-Installation selon l'une des revendications 3 à 6, caractérisée en ce que les sorties de condensat sont situées à un niveau supérieur au niveau d'introduction de ceux-ci (en 20) dans la colonne de distillation (8) pour assurer une alimentation par gravité.

8.-Installation selon l'une des revendications 3 à 7, caractérisée en que chaque pression de détente et en particulier la pression de détente ultime est choisie à une valeur telle qu'il existe un écart de pression suffisant pour que le liquide puisse circuler sans pompe.

9.-Installation selon l'une des revendications 3 à 8, caractérisée en ce que la solution liquide de xylose est choisie parmi une pâte papetière ou de préférence une pâte textile.

0 211 713

## Office européen des brevets

## RAPPORT DE RECHERCHE EUROPEENNE

Numero de la demande

EP 86 40 1450

### DOCUMENTS CONSIDERES COMME PERTINENTS

| Catégorie | Citation du document avec indication. en cas de besoin. des parties pertinentes | Revendication concernee | CLASSEMENT DE LA DEMANDE (Int Cl 4) |
|---|---|---|---|
| D,Y | FR-A-1 510 864 (SICAMIE)<br>* En entier * | 1-9 | C 07 D 307/50 |
| Y | FR-A-1 176 060 (TERMODINAMICA)<br>* En entier * | 1-9 | |
| Y | DE-A-2 250 548 (T. BEYE)<br>* En entier * | 1-9 | |
| Y | US-A-2 350 584 (C.K. BUELL)<br>* En entier, en particulier figure 2 * | 1-9 | |

---

DOMAINES TECHNIQUES
RECHERCHES (Int. Cl.4)

C 07 D 307/00

Le présent rapport de recherche a été établi pour toutes les revendications

| Lieu de la recherche | Date d'achèvement de la recherche | Examinateur |
|---|---|---|
| LA HAYE | 14-10-1986 | ALLARD M.S. |